# FASCICULE DE BREVET EUROPEEN

(11) **EP 2 732 786 B1**
(45) Date de publication et mention de la délivrance du brevet: **07.03.2018**
(21) Numéro de dépôt: 13192136.3
(22) Date de dépôt: 08.11.2013
(51) Int. Cl.: A61B 18/20, A61B 18/00

(54) **Appareil de traitement de la peau equipé d'un moyen de guidage**
Hautbehandlungsgerät, das mit einem Führungsmittel ausgestattet ist
Skin treatment apparatus provided with a guiding means

(30) Priorité: 19.11.2012 FR 1260991
(43) Date de publication de la demande: 21.05.2014
(73) Titulaire: SEB S.A., 69130 Ecully (FR)
(72) Inventeur: Chambon, Vincent, 69510 Soucieu en Jarrest (FR); Maisonneuve, Martial, 38090 Villefontaine (FR); Vacheron, Xavier, 69740 Genas (FR)
(74) Mandataire: Guéry-Jacques, Géraldine

(56) Documents cités:
- WO-A1-01/26573
- WO-A1-2006/092776
- US-B1- 6 171 302

## Description

La présente invention concerne le domaine des appareils de traitement moyennant une fenêtre de traitement pour traiter la peau au travers de cette fenêtre et un moyen de guidage pour venir en aide à l'utilisateur pendant le traitement pour déplacer l'appareil sur la peau pour obtenir un traitement optimal et efficace. L'invention concerne particulièrement les appareils d'épilation par sources lumineuses.

Des appareils d'épilation par lumière pulsée sont déjà connus aujourd'hui par leur fonction d'éliminer les poils sur la peau et de prévenir leur repousse. Un tel appareil est équipé d'une fenêtre de traitement ayant une forme souvent rectangulaire par laquelle un émetteur de lumière envoie des flashages sur la peau à traiter. Pour pouvoir traiter une zone entière, l'utilisateur doit déplacer cette fenêtre près de la peau d'un endroit à un autre pour effectuer plusieurs flashages sans laisser d'indication visible sur la peau, puisque l'effet du traitement est constatable seulement quelques jours ou semaines après. De ce fait, l'utilisateur peut facilement oublier de traiter certaines parties de la zone ou répéter le traitement sur une partie déjà traitée, ce qui peut diminuer l'efficacité du traitement voire entrainer des conséquences non souhaitées sur la peau. Il est donc apparu le besoin d'un moyen de guidage pouvant guider l'utilisateur dans le déplacement de la fenêtre de flashage pour éviter de traiter une partie de la peau plus d'une fois tout en assurant un traitement exhaustif sur une zone voulue.

Pour arriver à ces objectifs, un document WO2006092776 décrit un appareil de traitement de la peau par lumière pulsée comprenant une fenêtre de traitement et un accessoire destiné à fournir une indication visuelle ou auditive sur la position de l'appareil au moyen d'un dispositif de mesure de position, associé avec un circuit de contrôle, le dispositif de mesure comprenant une roue codée ayant un périmètre correspondant à la largeur de la fenêtre de traitement. Pendant le traitement, l'utilisateur avance l'appareil sur la peau et entraine la rotation de la roue, l'accessoire envoie un signal à l'utilisateur pour qu'il effectue un flashage lorsque l'avancement d'un tour de la roue est détecté par le dispositif de mesure. Cette solution permet donc de traiter automatiquement une zone successivement sans avoir des endroits oubliés ou des endroits traités plusieurs fois. Néanmoins, l'accessoire décrit dans ce document implique des composants électroniques, donc augmente le coût du produit. De plus, il nécessite une alimentation électrique, ce qui complique l'usage et la maintenance du produit.

On connait un autre document WO2009/037641 qui décrit un appareil de traitement pour la peau comprenant une fenêtre de traitement et un moyen de guidage attaché à l'appareil pour guider l'utilisateur à faire un déplacement par à-coups. Le moyen de guidage comprend un rouleau à section polygonale définissant de chaque côté une largeur correspondant substantiellement à la largeur de la fenêtre de traitement. L'utilisateur prenant l'appareil et déplaçant la fenêtre le long de la peau reçoit et ressent un signal tactile provenant du rouleau et sait qu'il doit arrêter le déplacement de l'appareil pour effectuer un flashage. Ce document propose donc un accessoire totalement mécanique et d'une structure simple. Toutefois, la sensibilité d'un corps humain n'est pas la même selon les utilisateurs et les utilisateurs ne peuvent disposer de la même perception d'un signal tactile. Pour une personne non sensible aux signaux tactiles provenant d'un rouleau à section polygonale, cette solution pourrait être inefficace. De plus, tenant compte du fait que la forme et les dimensions du rouleau nécessitent une grande précision pour répondre à la fonctionnalité de l'accessoire, la fabrication de ce dernier est compliquée et coûteuse. WO01/26573 A1 divulgue un appareil de traitement comprenant un boîtier, une fenêtre de traitement et un moyen de guidage adjacent à la fenêtre de traitement destiné à fournir des indications sur le positionnement de l'appareil.

Le but de l'invention est de remédier aux inconvénients susmentionnés et de proposer un appareil de traitement de la peau équipé d'un moyen de guidage permettant de fournir des indications plus faciles à percevoir par l'utilisateur pendant le traitement.

Un autre de but de l'invention est de fournir un appareil de traitement de la peau équipé d'un moyen de guidage permettant un déplacement de l'appareil facile et fluide.

Un autre but de l'invention est de fournir un appareil de traitement de la peau équipé d'un moyen de guidage à faible coût.

Un autre but de l'invention est de fournir un appareil de traitement de la peau équipé d'un moyen de guidage facile à fabriquer.

Encore un autre but de l'invention est de fournir un appareil de traitement de la peau équipé d'un moyen de guidage permettant un traitement optimal et plus efficace, sans zone oubliée ou endommagée.

Ces buts sont atteints avec un appareil de traitement destiné à traiter successivement la peau dans une zone prédéterminée par un déplacement le long de cette zone, comprenant un boitier, une fenêtre de traitement d'une largeur I et comprise dans un plan (A), un moyen de guidage adjacent à la fenêtre de traitement destiné à fournir des indications sur le positionnement de l'appareil comprenant un corps solidaire au boitier et au moins un rouleau ayant un corps cylindrique dont l'axe de symétrie Δ est parallèle au plan de la fenêtre de traitement, le rouleau étant monté sur le corps et libre en rotation par rapport au corps. Selon l'invention, le rouleau présente au moins une zone principale et une zone secondaire adjacentes et visiblement distinctes et le corps comprend au moins une ouverture traversante en regard du rouleau, la taille de ladite ouverture étant dimensionnée pour permettre d'afficher alternativement la zone principale indiquant à l'utilisateur que l'appareil arrive à une position de traitement souhaitée et au moins une partie de la zone secondaire indiquant à l'utilisateur que l'appareil est hors d'une position de traitement souhaitée.
La mise en place du rouleau permet de guider l'utilisateur à déplacer l'appareil de traitement en ligne droite, donc le déplacement de l'appareil est plus intuitif et fluide. De plus, les zones visiblement distinctes et adjacentes étant rotatives avec le mouvement du rouleau, elles sont affichées et visualisées par l'utilisateur au travers de ladite ouverture traversante de façon alternative sur la ligne de déplacement afin d'indiquer à l'utilisateur le positionnement de l'appareil. On comprend par ladite position de traitement souhaitée une position dans laquelle la fenêtre de traitement se trouve juste au dessus d'une surface non traitée mais substantiellement adjacente à une autre surface venant d'être traitée précédemment. Quand l'appareil est en position de traitement souhaitée, l'utilisateur peut être incité à arrêter ou décélérer le déplacement de l'appareil et effectue un traitement ; dans le cas contraire, l'utilisateur est amené à continuer à déplacer l'appareil jusqu'à ce que l'appareil arrive à nouveau à une position de traitement souhaitée.

Par ailleurs, on comprend par zones visiblement distinctes des zones comprenant des couleurs et/ou motifs différents permettant à l'utilisateur de distinguer visuellement la zone principale et la zone secondaire. Ainsi, l'utilisateur perçoit facilement et rapidement le changement d'une zone à une autre dans l'ouverture traversante.

De préférence, lesdites zones sont agencées au moins sur les bases du rouleau. Ceci permet une facilité de visualisation des zones car les bases restent visibles alors que la courbe directrice du rouleau est en contact avec la peau le long du déplacement.

Avantageusement, le rouleau comprend à chacune de ses extrémités une roue codée ayant un corps tubulaire coaxial avec le rouleau et au moins une face circulaire comprise dans la base du rouleau, ladite roue codée étant liée en rotation avec le corps cylindrique et amovible en translation sur l'axe Δ. Les roues codée facilitent l'installation du rouleau sur le corps ainsi que l'intégration des zones dans le rouleau. Le rouleau peut avoir une forme de cylindre de révolution, ce qui permet un déplacement le long de la peau le plus fluide possible, sans à-coups.

Selon une variante, la face circulaire comprend en direction radiale trois zones principales espacées respectivement par trois zones secondaires, la zone principale ayant une répartition angulaire α, la zone secondaire ayant une répartition angulaire β. Le nombre des zones principales et secondaires a été calculé par rapport aux dimensions du rouleau. En modifiant ces dernières, on peut augmenter et diminuer le nombre des zones ainsi que leurs répartitions angulaires. Selon l'invention, la taille du rouleau est adaptée à l'ergonomie et aux dimensions de l'appareil de traitement. La répartition angulaire α dépend de la largeur de l'ouverture traversante et du périmètre du rouleau.

De surcroît, la roue codée présente une distance circonférentielle de deux zones adjacentes comprise entre 60% et 100% de la largeur I. Préférablement, cette distance correspond à une valeur au tour de 80% de la largeur I. Ceci présente une longueur de parcours entre deux traitements adjacents qui est légèrement plus réduite que la largeur I de la fenêtre de traitement. Ainsi, on crée une petite zone de chevauchement entre deux surfaces voisines traitées pour éviter toute surface manquante.

Dans une alternative, les zones sont prolongées depuis la face circulaire vers le corps cylindrique du rouleau. Ceci permet encore une facilité de visualisation des zones.

Selon une alternative, le moyen de guidage comprend sur ledit corps au moins une pièce transparente venant couvrir au moins une partie de ladite ouverture traversante. La pièce transparente peut être une lentille en verre ou en plastique donnant l'accès à la visualisation des zones et protégeant les composants du moyen de guidage des poussières et du choc.

Avantageusement, le rouleau comprend dans son corps cylindrique au moins un moyen de rappel destiné à écarter les roues codées contre le corps de l'accessoire. Ceci permet de faciliter le montage des roues codées et de les maintenir au plus près des ouvertures traversantes pour faciliter la visualisation des zones.

De surcroît, la surface cylindrique du rouleau destinée à entrer en contact avec la peau dépasse légèrement et vers l'extérieur hors du plan A de la fenêtre de traitement. Cette particularité permet d'obliger le contact entre le rouleau et la peau, même sur les zones galbées du corps humain.

Avantageusement, le rouleau comprend sur la surface de son corps cylindrique des moyens d'adhérence à la peau. On comprend par moyen d'adhérence toute utilisation de matériau ou de forme permettant d'augmenter le coefficient de frottement entre le rouleau et la peau pour assurer un bon entrainement en rotation du rouleau et s'opposer à un glissement. On peut très bien imaginer l'utilisation d'un matériau sur la surface du corps cylindrique du rouleau pour rendre celle-ci rugueuse, ou encore des demi sphères réparties sur cette surface. Le matériau peut être d'une base thermoplastique élastomère (du type : SEBS, TPE, PP Epdm, etc...)

De préférence, ledit moyen de guidage est détachable du boîtier. Ceci donne à l'utilisateur la possibilité de choisir la présence ou non du moyen de guidage, ou encore permet de nettoyer plus facilement entre deux traitements le rouleau et la fenêtre de traitement.

Selon l'invention, l'appareil comprend un émetteur de lumière et un bouton de commande pour déclencher ladite émission au travers de la fenêtre. Ceci donne à l'utilisateur la possibilité de commander lui-même le traitement.

Ces buts sont également atteints avec un procédé d'utilisation de l'appareil de traitement comprenant les étapes suivantes : appliquer constamment la fenêtre de traitement sur la peau et déplacer l'appareil sur la peau ; appuyer sur le bouton quand la zone principale est visible au travers de l'ouverture traversante; avancer l'appareil sans appuyer sur le bouton aussi longtemps que la zone secondaire est visible au travers de l'ouverture traversante et recommencer l'opération sur toute la zone de peau à traiter. L'utilisateur suit le chemin du mouvement de l'appareil défini par le moyen de guidage et répète ces étapes jusqu'à ce que la zone prédéterminée soit complètement traitée.

L'invention sera mieux comprise à l'étude d'un mode de réalisation pris à titre nullement limitatif et illustré dans les figures annexées dans lesquelles :
- La Figure 1 est une vue de face de l'ensemble de l'appareil de traitement avec le moyen de guidage;
- La Figure 2 est une vue de côté de l'ensemble de l'appareil de traitement avec le moyen de guidage ;
- La Figure 3 est une vue en perspective de l'ensemble du moyen de guidage ;
- La Figure 4 est une vue en perspective de l'ensemble du moyen de guidage éclaté ;
- La Figure 5 est une vue de coupe de l'ensemble du moyen de guidage ;
- La Figure 6 est une vue en perspective de la roue codée;
- La Figure 7 est une vue en perspective du corps cylindrique du rouleau.

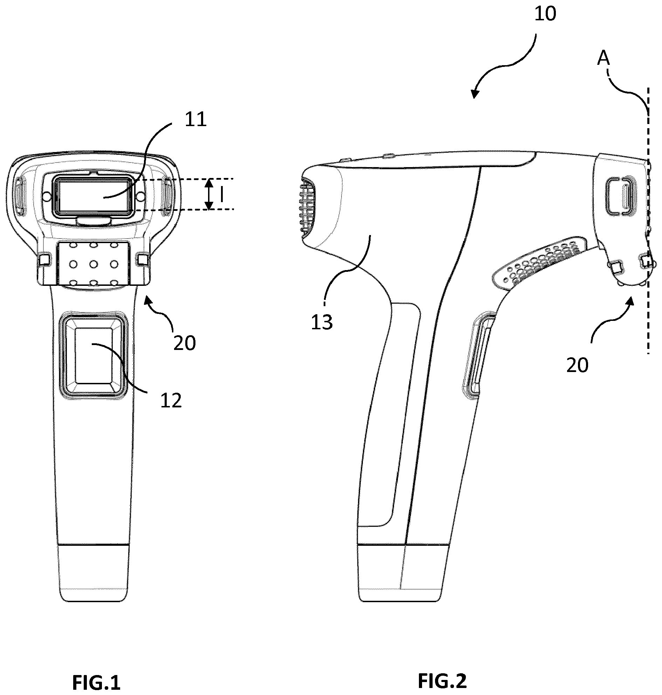

Un appareil de traitement pour la peau tel qu'illustré à la Figure 1 et désigné dans son ensemble par la référence 10 comprend un boîtier 13 définissant une zone de préhension et étant équipé sur la partie inférieure d'un bouton de commande 12 destiné par appui à déclencher un traitement au travers d'une fenêtre de traitement 11 d'une largeur I. On comprend par appareil de traitement tout type d'appareil permettant de fournir un traitement mécanique, optique ou chimique. La source lumineuse à lumière pulsée utilisée dans un appareil de traitement optique peut alternativement être une source lumineuse LASER ou des LED ou des infrarouges etc. Dans un exemple précis, l'invention concerne un appareil d'épilation par lumière pulsée, destiné à désactiver les follicules sous la peau pour éliminer les poils et prévenir ou retarder leur repousse. Pendant l'utilisation, l'utilisateur appuie sur le bouton 12 pour déclencher un flashage lors duquel des faisceaux lumineux sont émis vers la peau au travers de la fenêtre de traitement 11. Pour traiter une zone prédéterminée, par exemple le bras ou la jambe, l'utilisateur est amené à déplacer successivement l'appareil contre la peau et à effectuer un flashage à chaque position de traitement souhaitée définie plus haut.

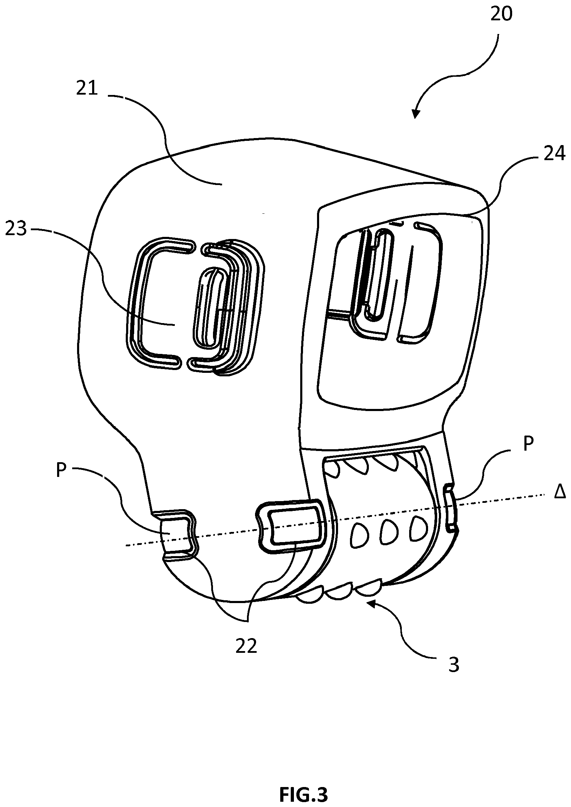

Tel que visible aux Figures 1 et 2, et selon une variante de l'invention, l'appareil comprend sur sa partie supérieure un moyen 20 de guidage sous forme d'un accessoire détachable du boîtier de l'appareil, tel qu'illustré à la Figure 3. L'accessoire 20 comprend un corps 21 en ABS et/ou PC présentant des moyens de fixation réversible 23 pour s'accrocher sur le boitier 13 de l'appareil. Le corps 21 comprend en outre une ouverture 24 venant se mettre autour de la fenêtre de traitement 11 lorsque l'accessoire 20 est attaché à l'appareil, les dimensions de cette ouverture 24 étant choisies de sorte que la fenêtre de traitement 11 soit complètement exposée par cette ouverture 24. On peut bien sur imaginer un moyen de guidage demeurant fixe par rapport au boîtier.

Le corps 21 comprend sur sa partie inférieure un rouleau 3 d'un axe de symétrie Δ monté sur le corps et libre en rotation par rapport au corps, l'axe Δ étant parallèle au plan de la fenêtre de traitement et perpendiculaire à l'axe de symétrie de l'appareil lorsque l'accessoire est monté sur le boîtier. Le rouleau 3 est adjacent à et en dessous de la fenêtre de traitement 11. Le rouleau destiné à guider l'utilisateur à déplacer l'appareil en ligne droite est rotatif dans un sens ou dans l'autre selon la direction du déplacement, la largeur I de la fenêtre étant sur cette ligne de déplacement. On peut envisager que le rouleau soit rotatif dans un sens unique de rotation pour guider l'utilisateur à appliquer dans l'ordre de déplacement d'abord le rouleau puis la fenêtre de traitement. Et enfin, l'accessoire 20 comprend sur le corps 21 des ouvertures traversantes 22 qui sont agencées en regard d'une partie du rouleau pour rendre le mouvement de celui-ci visible au travers de ces ouvertures traversantes 22. Dans une variante préférée de l'invention, les ouvertures traversantes 22 se trouvent en face des bases P du rouleau 3 pour que le déplacement du rouleau soit toujours visible que l'appareil soit tenu avec la main gauche ou la main droite ; elles sont légèrement prolongées de 3 à 5 mm vers le devant et l'arrière de l'accessoire pour élargir le champ de visualisation.

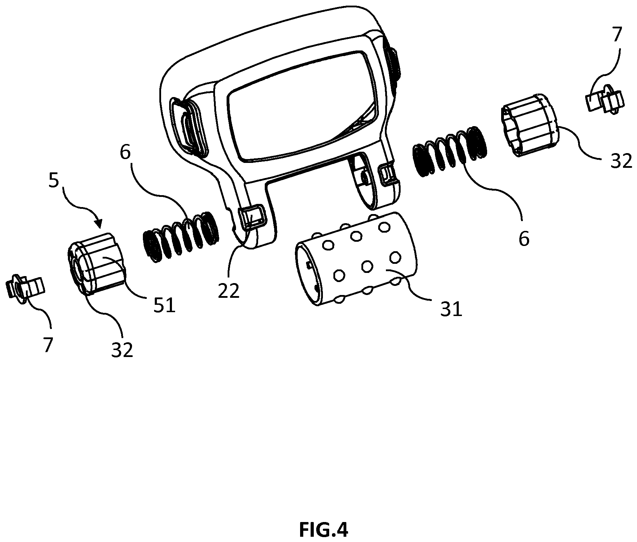

Selon une variante, et tel que visible à la Figure 4, l'accessoire comprend sur chaque coté du corps 21 une pièce transparente 7 sous forme d'une lentille en plastique découvrant les deux ouvertures 22 traversantes du même coté.

Maintenant on va présenter le rouleau 3 en détail. Le rouleau 3 a une forme cylindrique de révolution présentant deux bases P parallèles et un corps cylindrique 31 dont la longueur est comprise entre 20 mm et 60mm et dont le diamètre est comprise entre 5mm et 20mm. Selon un exemple de l'invention, la longueur et le diamètre du rouleau sont respectivement de 31mm et 19,9mm pour un résultat optimal. Le rouleau 3 comprend sur chacune de ses extrémités une roue codée 5 ayant un corps tubulaire 51 coaxial avec le rouleau 3 et au moins une face circulaire 32 comprise dans la base P du rouleau.

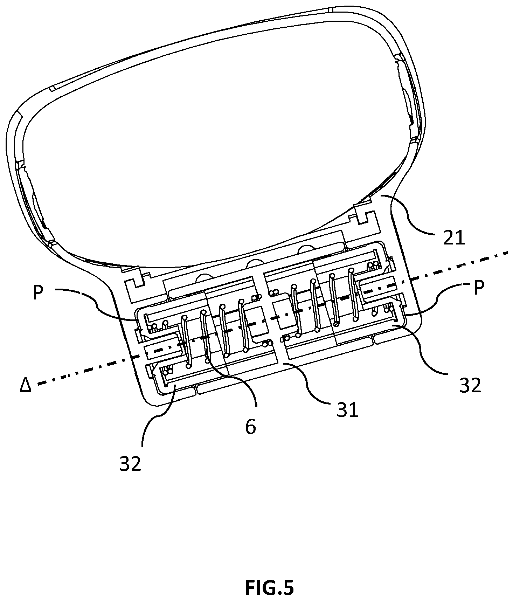

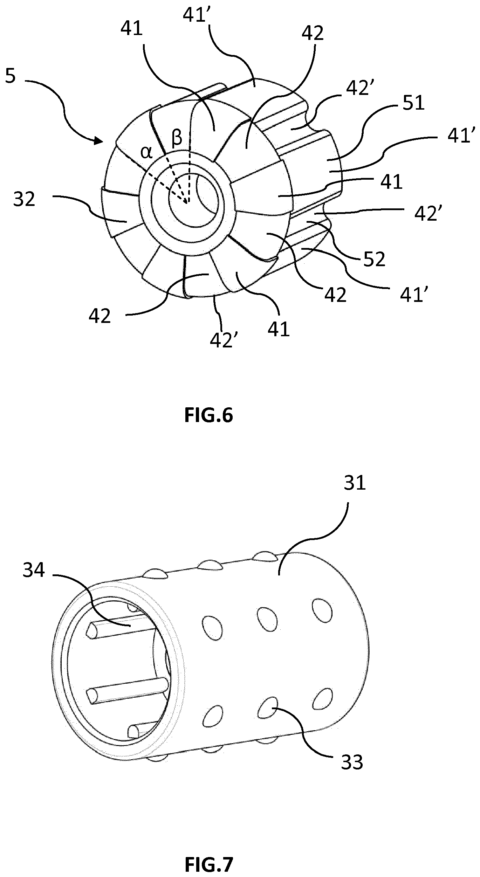

Comme illustrée aux figures 6 et 7, la roue codée 5 comprend sur son corps tubulaire 51 des rainures 52 parallèles à l'axe Δ destinées à recevoir des lignes en protubérance 34 sur la surface intérieure du corps cylindrique 31 du rouleau 3 pour que la roue codée et le corps cylindrique 31 soient liés en rotation. La face circulaire 32 de la roue codée est plus grande que celle du corps cylindrique 31 du rouleau et limite le mouvement de la roue codée 5 en translation par rapport au corps cylindrique 31. Tel que visible à la Figure 5, le rouleau 3 comprend dans son corps cylindrique 31 au moins un moyen de rappel 6 destiné à écarter les roues codées 5 contre le corps 21 de l'accessoire 20. Cette structure permet de garder les faces circulaires 32 toujours au plus près des ouvertures 22 traversantes. Elle permet également un montage facile de l'ensemble du rouleau 3.

Telle que visible à la Figure 6, la roue codée comprend sur sa face circulaire 32 en direction radiale trois zones principales 41 chacune ayant une répartition angulaire α, et trois zones secondaires 42 chacune ayant une répartition angulaire β, toutes les deux zones principales 41 étant espacées par une zone secondaire 42. Ceci a pour but d'afficher les zones principales 41, 41' et les zones secondaires 42, 42' dans l'ouverture 22 traversante de façon alternative. Une zone principale 41 et une zone secondaire 42 sont toujours adjacentes et visiblement distinctes pour que l'utilisateur puisse facilement observer le changement de zone dans l'ouverture 22. Pour que les zones principale et secondaire soient suffisamment distinctes, on peut utiliser des codes couleur pour que les deux zones 41, 42 aient des couleurs visiblement différentes. On peut aussi utiliser des motifs ou des finitions de surface sur chacune des zones 41, 42 aussi longtemps que la distinction visuelle des deux zones 41, 42 soit facile et évidente. Afin d'élargir le champ d'indication visuelle, les zones 41', 42' peuvent aussi prolonger depuis la face circulaire 32 vers le corps tubulaire 51 pour être visibles à l'avant et à l'arrière de l'accessoire 20. Et enfin, les zones 41, 41', 42, 42' sont agencées sur la surface externe du rouleau 3.

Les répartitions angulaires α et β ne sont pas obligatoirement identiques. Cependant, il est important que l'ensemble d'une zone principale 41 et d'une zone secondaire 42 voisines présente une distance circonférentielle adaptée au fonctionnement de l'accessoire. Pour ne pas avoir de surface manquante pendant le traitement, on crée volontairement un chevauchement compris entre 0%- 40% de la largeur I sur deux zones voisines traitées. Cette valeur est idéalement au tour de 20%. De ce fait, la distance circonférentielle de deux zones voisine 41 et 42 correspond à substantiellement 80% de la larguer I de la fenêtre.

Dans l'utilisation, l'utilisateur déplace l'appareil contre la peau et appuie sur le bouton quand la zone principale est visible au travers de l'ouverture ; l'utilisateur avance l'appareil sans appuyer sur le bouton aussi longtemps que la zone secondaire est visible au travers de l'ouverture et veille à ce que la zone principale s'affiche de nouveau pour appuyer sur le bouton. L'utilisateur suit le chemin du mouvement de l'appareil défini par le moyen de guidage et répète ces étapes jusqu'à ce que la zone prédéterminée soit complètement traitée.

On peut aussi utiliser un rouleau monobloc contenant les zones 41, 41', 42, 42' sur les deux bases du corps cylindrique, cependant, cette solution est plus compliquée en fabrication et en montage sur le corps de l'accessoire.

Dans le but d'augmenter le coefficient de frottement du rouleau 3 sur la peau pour que celui-ci soit plus facile à entrainer sans glissement intempestif, le corps cylindrique 31 du rouleau 3 est fabriqué d'une matière souple et comprend sur la surface des moyens d'adhérence 33 à la peau sous forme de demi-sphères.

Bien entendu, d'autres modifications peuvent être apportées à l'invention dans le cadre des revendications annexées.

## Revendications

1. Appareil (10) de traitement destiné à traiter successivement la peau dans une zone prédéterminée par un déplacement le long de cette zone, comprenant
- un boitier (13)
- une fenêtre de traitement (11) d'une largeur I et comprise dans un plan (A),
- un moyen (20) de guidage adjacent à la fenêtre de traitement (11) destiné à fournir des indications sur le positionnement de l'appareil (10) comprenant
- un corps (21) accroché de manière solidaire au boitier et
- au moins un rouleau (3) ayant un corps cylindrique (31) dont l'axe de symétrie Δ est parallèle au plan de la fenêtre de traitement (11), le rouleau étant monté sur le corps (21) et libre en rotation par rapport au corps (21),
**caractérisé en ce que** :
Le rouleau présente au moins une zone principale (41, 41') et une zone secondaire (42, 42') adjacentes et visiblement distinctes,
et **en ce que** :
le corps (21) comprend au moins une ouverture (22) traversante en regard du rouleau agencée pour rendre le mouvement du rouleau visible au travers de ladite ouverture traversante (22), la taille de ladite ouverture (22) étant dimensionnée pour permettre d'afficher alternativement la zone principale (41, 41') indiquant à l'utilisateur que l'appareil arrive à une position de traitement souhaitée et au moins une partie de la zone secondaire (42, 42') indiquant à l'utilisateur que l'appareil est hors d'une position de traitement souhaitée.

2. Appareil selon la revendication précédente, **caractérisé en ce que** lesdites zones (41, 41', 42, 42') sont agencées au moins sur les bases (P) du rouleau.

3. Appareil selon la revendication précédente, **caractérisé en ce que** le rouleau comprend à chacune de ses extrémités une roue codée (5) ayant un corps tubulaire (51) coaxial avec le rouleau (3) et au moins une face circulaire (32) comprise dans la base (P) du rouleau, ladite roue codée (5) étant liée en rotation avec le corps cylindrique (31) et amovible en translation sur l'axe Δ.

4. Appareil selon la revendication précédente, **caractérisé en ce que** la face circulaire (32) comprend en direction radiale trois zones principales (41, 41') espacées respectivement par trois zones secondaires (42, 42'), la zone principale (41, 41') ayant une répartition angulaire α, la zone secondaire (42, 42') ayant une répartition angulaire β.

5. Appareil selon la revendication précédente, **caractérisé en ce que** la roue codée (5) présente une distance circonférentielle de deux zones adjacentes (41, 41', 42, 42') comprise entre 60% et 100% de la largeur I.

6. Appareil selon une des revendications 3 à 5, **caractérisé en ce que** les zones (41, 41', 42, 42') sont prolongées depuis la face circulaire (32) vers le corps cylindrique (31) du rouleau.

7. Appareil selon une des revendications précédentes, **caractérisé en ce que** le moyen (20) de guidage comprend sur ledit corps (21) au moins une pièce transparente (7) venant couvrir au moins une partie de ladite ouverture (22) traversante.

8. Appareil selon une des revendications 3 à 7, **caractérisé en ce que** le rouleau (3) comprend dans son corps cylindrique (31) au moins un moyen de rappel (6) destiné à écarter les roues codées (5) contre le corps (21) de l'accessoire (20).

9. Appareil selon une des revendications précédentes, **caractérisé en ce que** la surface cylindrique du rouleau destinée à entrer en contact avec la peau dépasse légèrement et vers l'extérieur hors du plan (A) de la fenêtre de traitement (11).

10. Appareil selon une des revendications précédentes, **caractérisé en ce que** le rouleau (3) comprend sur la surface de son corps cylindrique (31) des moyens d'adhérence (33) à la peau.

11. Appareil selon une des revendications précédentes, **caractérisé en ce que** ledit moyen (20) de guidage est détachable du boîtier (13).

12. Appareil selon une des revendications précédentes, **caractérisé en ce qu'**il comprend un émetteur de lumière et un bouton de commande (12) pour déclencher ladite émission au travers de la fenêtre.

## Patentansprüche

1. Gerät (10) zur Behandlung, das dazu bestimmt ist, sukzessive die Haut in einem vorbestimmten Bereich durch eine Bewegung entlang dieses Bereichs zu behandeln, umfassend:
- ein Gehäuse (13),
- ein Behandlungsfenster (11) mit einer Breite I, das in einer Ebene (A) liegt,
- ein Mittel (20) zur Führung neben dem Behandlungsfenster (11), das dazu bestimmt sind, Angaben über die Positionierung des Geräts (10) zu liefern, umfassend
- einen Körper (21), der fest an dem Gehäuse angebracht ist, und
- mindestens eine Rolle (3), die einen zylindrischen Körper (31) hat, dessen Symmetrieachse Δ parallel zu dem Behandlungsfenster (11) ist, wobei die Rolle auf dem Körper (21) angebracht ist und relativ zu dem Körper (21) frei drehbar ist,
**dadurch gekennzeichnet, dass**:
die Rolle mindestens einen primären Bereich (41, 41') und eine sekundären Bereich (42, 42') aufweist, die benachbart und sichtbar verschieden sind,
und dass:
der Körper (21) mindestens eine durchgehende Öffnung (22) gegenüber der Rolle umfasst, die eingerichtet ist, die Bewegung der Rolle durch die durchgehende Öffnung (22) sichtbar zu machen,
wobei die Größe der Öffnung (22) so dimensioniert ist, um es zu ermöglichen, alternativ die Primärzone (41, 41'), die dem Benutzer indiziert, dass das Gerät eine gewünschte Behandlungsposition erreicht hat, und mindestens einen Abschnitt des sekundären Bereichs (42, 42') anzuzeigen, der dem Benutzer indiziert, dass sich das Gerät außerhalb einer gewünschten Behandlungsposition befindet.

2. Gerät nach dem vorhergehenden Anspruch, **dadurch gekennzeichnet, dass** die Bereiche (41, 41', 42, 42') mindestens an den Basen (P) der Rolle angeordnet sind.

3. Gerät nach dem vorhergehenden Anspruch, **dadurch gekennzeichnet, dass** die Rolle an jedem ihrer Enden ein kodiertes Rad (5) umfasst, das einen rohrförmigen Körper (51), der koaxial zu der Walze (3) ist, und mindestens eine kreisförmige Fläche (32) hat, die in der Basis (P) der Rolle liegt, wobei das kodierte Rad (5) drehbar mit dem zylindrischen Körper (31) verbunden ist und in Translation auf der Achse Δ beweglich ist.

4. Gerät nach dem vorhergehenden Anspruch, **dadurch gekennzeichnet, dass** die kreisförmige Fläche (32) in radialer Richtung drei primäre Bereiche (41, 41') umfasst, die jeweils durch drei sekundäre Bereiche (42, 42') beabstandet sind, wobei die primären Bereiche (41, 41') eine Winkelverteilung α haben, wobei die sekundären Bereiche (42, 42') eine Winkelverteilung β haben.

5. Gerät nach dem vorhergehenden Anspruch, **dadurch gekennzeichnet, dass** das codierte Rad (5) einen umlaufenden Abstand zwischen zwei benachbarten Bereichen (41, 41', 42, 42') aufweist, der zwischen 60% und 100% der Breite I liegt.

6. Gerät nach einem der Ansprüche 3 bis 5, **dadurch gekennzeichnet, dass** die Bereiche (41, 41', 42, 42') von der kreisförmigen Fläche (32) zu dem zylindrischen Körper (31) der Rolle verlängert sind.

7. Gerät nach einem der vorhergehenden Ansprüche, **dadurch gekennzeichnet, dass** das Mittel (20) zur Führung auf dem Körper (21) mindestens ein transparentes Teil (7) umfasst, das mindestens einen Abschnitt der durchgehenden Öffnung (22) abdeckt.

8. Gerät nach einem der Ansprüche 3 bis 7, **dadurch gekennzeichnet, dass** die Rolle (3) in ihrem zylindrischen Körper (31) mindestens ein Rückstellmittel (6) umfasst, das dazu bestimmt ist, die kodierten Räder (5) gegen den Körper (21) des Zubehörteils (20) abzuspreizen.

9. Gerät nach einem der vorhergehenden Ansprüche, **dadurch gekennzeichnet, dass** die zylindrische Oberfläche der Rolle, die dazu bestimmt ist, mit der Haut in Kontakt zu kommen, etwas nach außen von der Ebene (A) des Behandlungsfensters (11) hervorragt.

10. Gerät nach einem der vorhergehenden Ansprüche, **dadurch gekennzeichnet, dass** die Rolle (3) auf der Oberfläche ihres zylindrischen Körpers (31) Mittel zum Haften (33) an der Haut umfasst.

11. Gerät nach einem der vorhergehenden Ansprüche, **dadurch gekennzeichnet, dass** das Mittel (20) zur Führung von dem Gehäuse (13) abnehmbar ist.

12. Gerät nach einem der vorhergehenden Ansprüche, **dadurch gekennzeichnet, dass** es einen Lichtsender und einen Steuerknopf (12) zum Auslösen der Übertragung durch das Fenster umfasst.

## Claims

1. Treatment device (10) for sequentially treating the skin in a predetermined area via a movement over this area, comprising
- a housing (13)
- a treatment window (11) with a width I and included in a plane (A),
- a guide means (20) adjacent to the treatment window (11) and designed to provide information on the positioning of the device (10) and comprising
- a body (21) securely attached to the housing and
- at least one roller (3) having a cylindrical body (31) of which the axis of symmetry Δ is parallel to the plane of the treatment window (11), the roller being mounted on the body (21) and freely rotating relative to the body (21),
**characterized in that**:
The roller has at least one main area (41, 41') and a secondary area (42, 42') adjacent to and visibly distinct from one another,
and **in that**:
the body (21) comprises at least one through-opening (22) in relation to the roller arranged so as to render the movement of the roller visible through said opening (22) with the size of said opening (22) being dimensioned so as to display in an alternating manner the main area (41, 41') indicating to the user that the device has reached a desired treatment position and at least a portion of the secondary area (42, 42') indicating to the user that the device is beyond a desired treatment position.

2. Device as in the previous claim, **characterized in that** said areas (41, 41', 42, 42') are arranged at least on the bases (P) of the roller.

3. Device as in the previous claim, **characterized in that** the roller has on each of its ends a coded wheel (5) having a tubular body (51) coaxial with the roller (3) and at least one circular face (32) included in the base (P) of the roller, said coded wheel (5) being connected for rotation with the cylindrical body (31) and removable in translation on the axis Δ.

4. Device as in the previous claim, **characterized in that** the circular face (32) has in the radial direction three main areas (41, 41') spaced respectively by three secondary areas (42, 42'); the main area (41, 41') having an angular distribution α, the secondary area (42, 42') having an angular distribution β.

5. Device as in the previous claim, **characterized in that** the coded wheel (5) has a circumferential distance of two adjacent areas (41, 41', 42, 42') ranging from 60% to 100% of the width I.

6. Device as in one of claims 3 through 5, **characterized in that** the areas (41, 41', 42, 42') are extended from the circular face (32) to the cylindrical body (31) of the roller.

7. Device as in one of the previous claims, **characterized in that** the guide means (20) has on said body (21) at least one transparent piece (7) covering at least a portion of said through-opening (22).

8. Device as in one of claims 3 through 7, **characterized in that** the roller (3) has in its cylindrical body (31) at least one spring means (6) for moving the coded wheels (5) against the body (21) of the accessory (20).

9. Device as in one of the previous claims, **characterized in that** the cylindrical surface of the roller intended to come into contact with the skin extends slightly beyond the plane (A) of the treatment window (11) and to the outside.

10. Device as in one of the previous claims, **characterized in that** the roller (3) has means (33) for gripping the skin on the surface of its cylindrical body (31).

11. Device as in one of the previous claims, **characterized in that** said guide means (20) is detachable from the housing (13).

12. Device as in one of the previous claims, **characterized in that** it comprises a light emitter and a control button (12) for triggering said emission through the window.
